# EUROPÄISCHE PATENTANMELDUNG

(11) **EP 1 683 542 A1**
(43) Veröffentlichungstag der Anmeldung: **26.07.2006**
(21) Anmeldenummer: 05001046.1
(22) Anmeldetag: 19.01.2005
(51) Int. Cl.: A61M 39/10

(54) **Kanülenansatzsystem**

(71) Anmelder: Möller Medical GmbH & Co.KG, 36043 Fulda (DE)
(72) Erfinder: Herget, Bernhard, 36115 Ehrenberg (DE); Schäfer, Siegfried, 36100 Petersberg (DE); Knacker, Peter, 36115 Ehrenberg (DE)
(74) Vertreter: Grünecker, Kinkeldey, Stockmair & Schwanhäusser Anwaltssozietät

(57) **Zusammenfassung**

Durch die Erfindung soll ein Kanülenansatzsystem (1) mit Kanülenansatz (2) und einem weiteren Ansatz (4), welche über eine Verdrehsicherung (14) lösbar miteinander verbunden sind, dahingehend verbessert werden, dass der weitere Ansatz bei einfacher Handhabung am Kanülenansatz lösbar, aber gegen ungewolltes selbsttätiges Herausgleiten aus der Kanüle gesichert befestigbar ist, und das System trotzdem einfach zu fertigen ist. Dies wird mit einem erfindungsgemäßen Kanülenansatzsystem erreicht, bei welchem einer der Ansätze mindestens einen radialen Vorsprung (8) aufweist und der Andere Ansatz mindestens einen radialen Absatz (11) aufweist, wobei die Ansätze axial zusammenführbar sind und durch Verdrehen in einen lösbaren, bajonettartigen Hintergriff des Vorsprungs mit dem Absatz bringbar sind, wobei das Verdrehen in die Hintergriffstellung die Verdrehsicherung aktiviert.

## Beschreibung

Die Erfindung betrifft ein Kanülenansatzsystem mit den Merkmalen des Oberbegriffs des Anspruchs 1.

Ein aus der G 93 10 484.7 bekanntes Kanülenansatzsystem dieser Gattung besteht aus einem Kanülenansatz, welcher in seinem Rand eine Ausnehmung aufweist, und einem Mandrinansatz, welcher einen axial ausgerichteten Positioniervorsprung aufweist. Der Positioniervorsprung ist durch einen mittig verlaufenden Schlitz in zwei leicht ausgestellte Federarme unterteilt. Nach dem axialen Einführen des Positioniervorsprungs in die Ausnehmung soll dadurch ein ungewolltes Herausgleiten des Mandarins aus der Kanüle verhindert werden.

Um die durch die Federarme erzeugte Reibung genau so groß zu gestalten, dass ein selbsttätiges Lösen des Mandrins verhindert und gleichzeitig ein willentliches Herausziehen des Mandrins aus der Kanüle jederzeit leicht möglich ist, sind sehr enge Fertigungstoleranzen nötig, welche verhältnismäßig hohe Produktionskosten nach sich ziehen. Diese geringen Toleranzen bergen gleichzeitig das Risiko, dass schon durch geringen Verzug, z.B. durch Temperatureinflüsse, der Mandrin selbsttätig aus der Kanüle gleiten kann. Zudem gestaltet sich das Hineinfinden des Positioniervorsprungs in die korrespondierende Ausnehmung schwierig, da hierfür eine bestimmte Winkelposition des Mandrinansatzes gegenüber dem Kanülenansatz nötig ist, deren Auffinden nicht durch Positionierhilfen (z.B. Führungen) erleichtert wird, sondern dem Anwender selbst überlassen bleibt.

Der Erfindung liegt die Aufgabe zugrunde, ein Kanülenansatzsystem der eingangs genannten Gattung dahingehend zu verbessern, dass der weitere Ansatz bei einfacher Handhabung am Kanülenansatz lösbar, aber gegen ungewolltes selbsttätiges Herausgleiten aus der Kanüle gesichert befestigbar ist, und trotzdem einfach zu fertigen ist.

Diese Aufgabe wird erfindungsgemäß gelöst mit einem Kanülenansatzsystem mit den Merkmalen des Anspruchs 1.

Durch axiales Zusammenführen des Kanülenansatzes mit dem weiteren Ansatz wird der radiale Vorsprung in den weiteren Ansatz eingeführt. Zugleich wird die Verdrehsicherung durch das Anlegen des weiteren Ansatzes an dem Kanülenansatz vorgespannt. Durch anschließendes Verdrehen des weiteren Ansatzes gegenüber dem Kanülenansatz hintergreift der radiale Vorsprung den radialen Absatz des weiteren Ansatzes bajonettartig. Ist die Endposition dieses Hintergriffs erreicht, wird die Verdrehsicherung aktiviert und die Drehbewegung damit beendet.

Durch das bajonettartige Hintergreifen des radialen Vorsprungs mit dem radialen Absatz wird eine lösbare axiale Fixierung des weiteren Ansatzes und damit des Instruments, welches er trägt, in der Kanüle erreicht. Das Aktivieren der Verdrehsicherung sichert diese erste, axiale Sicherung zusätzlich gegen Lösen.

In einer vorteilhaften Ausführung der Erfindung kann die Verdrehsicherung als Raste ausgebildet sein. Die Raste ist in der verrasteten Stellung eine wirksame Sicherung, die nur mit bestimmtem Kraftaufwand lösbar ist, und damit den bajonettartigen Hintergriff sichert.

In einer günstigen Ausführungsform der Erfindung kann die Verdrehsicherung axial federnd ausgebildet sein. Durch die Federkraft, welche quer zu der Verdrehrichtung wirkt, wird der radiale Vorsprung beim Hintergreifen des radialen Absatzes gegen diesen gedrückt, wodurch ein Widerlager für das fedemde Element entsteht.

Bei einer besonderen Variante der Erfindung kann die Verdrehsicherung einen Federsteg aufweisen, welcher mit Hilfe einer Nut gebildet ist, die in geringem Abstand zur steckseitigen Stirnseite des Ansatzes, benachbart zu dem Federsteg vorgesehen ist. Durch die Nut wird ein Ausweichraum für die Rastnase der Verdrehsicherung gebildet, so dass die Rastnase während des Verdrehens der Ansätze gegeneinander federnd vorgespannt wird und somit ein gutes Einrasten der Rastnase in die korrespondierende Ausnehmung gewährleistet ist.

In besonderer Weise kann der Federsteg in die steckseitige Stirnfläche des Ansatzes integriert sein. Dies führt zu einer besonders kompakten Bauweise, die einfach herzustellen ist.

Bei einer vorteilhaften Ausführung ist der weitere Ansatz als Mandrin- oder Kanülenansatz ausgebildet. Dadurch lässt sich der Kanülenansatz sowohl mit einem Mandrinansatz als auch mit einem weiteren Kanülenansatz gut und sicher verbinden.

Im Folgenden wird eine Ausführungsform der Erfindung anhand der folgenden Zeichnungen beschrieben. Dabei zeigen:
- Figur 1: ein erfindungsgemäßes Kanülenansatzsystem, bei welchem der weitere Ansatz als Mandrinansatz ausgeführt ist, mit eingeführtem Mandrindraht, wobei der Kanülenansatz und der Mandrinansatz beabstandet sind,
- Figur 2: das System von Figur 1, wobei der Kanülenansatz und der Mandrinansatz separat nebeneinander dargestellt sind, und
- Figur 3: einen Teilausschnitt des erfindungsgemäßen Kanülenansatzsystems, wobei der Mandrinansatz mit dem Kanülenansatz verbunden ist.

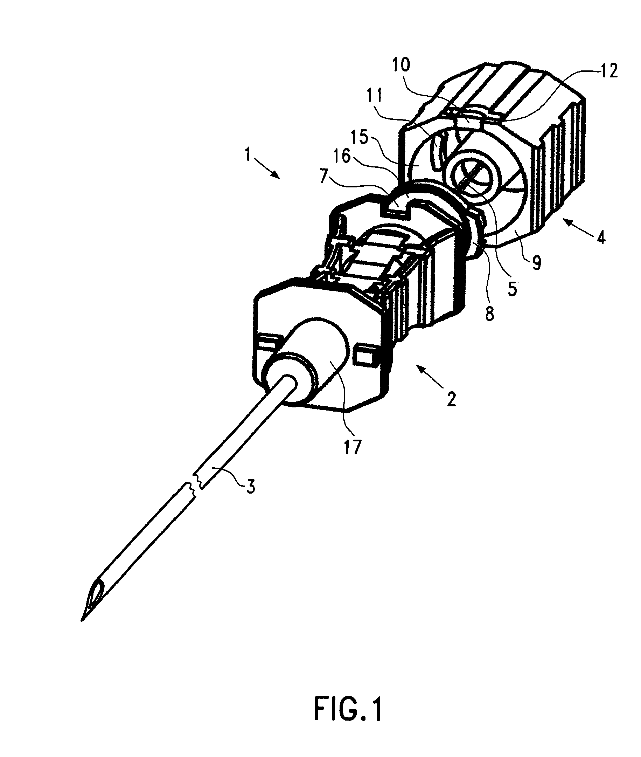

Figur 1 zeigt ein Kanülenansatzsystem 1 mit einem Kanülenansatz 2 mit einer Kanüle 3 und einem weiteren Ansatz, welcher hier als Mandrinansatz 4 mit einem Mandrin 5 ausgeführt ist, wobei der Mandrin bereits teilweise axial in die Kanüle 3 eingeführt ist. Der weitere Ansatz kann aber auch als Kanülenansatz ausgeführt sein. Der Kanülenansatz 2 weist an seinem der Kanüle gegenüberliegenden Ende eine steckseitige Stirnseite 6 auf, in welcher eine Ausnehmung 7 vorgesehen ist. Ausgehend von dieser Stirnseite 6 erstreckt sich ein rohrartiges Element 16, an dessen vorderem Ende sich zwei radiale Vorsprünge 8 in diametral symmetrischer Anordnung befinden. Das steckseitige Ende des Kanülenansatzes entspricht einem weiblichen Teil einer Luer-Lock-Verbindung, welches an seinem vorderen Ende die radialen Vorsprünge 8 trägt. Dadurch bildet es zusammen mit dem männlichen Teil 17, welches sich an dem gegenüberliegenden Ende des Kanülenansatzes befindet ein Luer-Lock. Dies ist dann von Vorteil, wenn der Kanülenansatz 2 mit einem weiteren Kanülenansatz bzw. einem weiteren Ansatz, welcher ebenfalls diese Schnittstelle bietet kombiniert werden soll.

Der Mandrinansatz 4 weist eine vordere, steckseitige Stirnfläche 9 auf, auf welcher sich eine mit der Ausnehmung 7 korrespondierende Rastnase 10 befindet. Ausgehend von der Stirnfläche 9 erstreckt sich eine Ausnehmung 15 in den Mandrinsatz 4. Die Ausnehmung ist als etwa zylindrischer Hohlraum 15 im Inneren des Mandrinansatzes ausgebildet. An der Innenwandung des Hohlraums befinden sich zwei symmetrisch angeordnete radiale Absätze 11, welche einteilig mit der Innenwand ausgeformt sind.

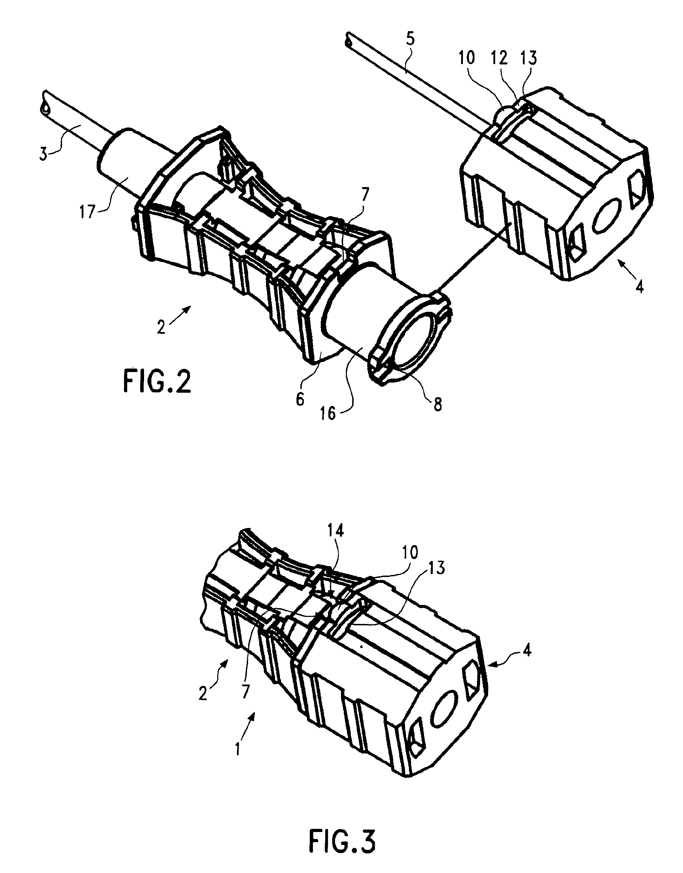

Figur 2 zeigt den Mandrinansatz 4 und den Kanülenansatz 2 in einem voneinander beabstandeten Zustand. Die in Figur 1 verwendeten Bezugszeichen bezeichnen dieselben Teile wie in Figur 2, so dass diesbezüglich auf die Beschreibung von Figur 1 verwiesen wird. Der Mandrinansatz 4 weist einen einteilig mit seiner steckseitigen Stirnfläche 9 ausgebildeten Federsteg 12 auf, welcher die Rastnase 10 trägt. Benachbart zu dem Federsteg 12 befindet sich eine Nut 13. Der Federsteg 12 und die Nut 13 erstrecken sich jeweils etwa senkrecht zu der axialen Erstreckungsrichtung der Kanüle 3 und des Mandrins 5 bzw. zu der axialen Zusammenführrichtung beider Ansätze.

Figur 3 zeigt den Mandrinansatz 4 in mit dem Kanülenansatz 2 verbundenem Zustand. In diesem Zustand liegt die Stirnfläche des Kanülenansatzes 6 direkt an der Stirnfläche des Mandrinansatzes 9 an. Das rohrartige Element 16 erstreckt sich dabei in den zylindrischen Hohlraum 15 im Inneren des Mandrinansatzes 4 und die radialen Vorsprünge 8 am vorderen Ende des rohrartigen Elementes 16 hintergreifen die radialen Absätze 11. Gleichzeitig befindet sich die Verdrehsicherung 14, welche aus der Rastnase 10 und der Ausnehmung 7 gebildet wird, in eingerastetem Zustand.

Im Folgenden wird die Funktionsweise der in den Figuren 1 bis 3 dargestellten erfindungsgemäßen Ausführungsform erläutert.

Durch axiales Einführen des Mandrins 5 in die Kanüle 3 und Aufschieben des Mandrinansatzes 4 auf den Kanülenansatz 2 werden die radialen Vorsprünge 8 in den Hohlraum 15 des Mandrinansatz 4 eingeführt. Dabei sind die Ansatzstücke 2, 4 derart gegeneinander verdreht, dass die radialen Vorsprünge 8 beim axialen Einführen in den Mandrinansatz 4 nicht mit den radialen Absätzen 11 kollidieren, sondern an diesen vorbei gleiten.

Dann liegt die steckseitige Stirnseite des Mandrinansatzes 9 an der steckseitigen Stirnseite des Kanülenansatzes 6 an, wodurch eine federnde Vorspannung der Rastnase 10 erzeugt wird. In dieser Position befinden sich der Mandrinansatz 4 und der Kanülenansatz 2 noch in beliebiger Winkelposition zueinander. Durch Verdrehen der Ansätze gegeneinander hintergreifen die radialen Vorsprünge 8 die radialen Absätze 11 im Inneren des Mandrinansatzes 4 bajonettartig. Bei Erreichen der endgültigen Winkelposition, rastet die Rastnase 10 in die Ausnehmung 7 ein. Zum einen wird dem Anwender dadurch das Finden der endgültigen Winkelposition erleichtert sowie das Erreichen der Endlage durch ein spürbares Einrasten signalisiert. Zum anderen wird der Mandrinansatz 4 durch das Einrasten der Rastnase 10 in die Ausnehmung 7 gegen ein versehentliches, ungewolltes Verdrehen gegenüber dem Kanülenansatz 2 und der Bajonettverschluss damit gegen Lösen gesichert. Dadurch wird eine axiale Fixierung des Mandrins 5 bzw. des Mandrinansatzes 4 gegenüber dem Kanülenansatz 2 erreicht, so dass der Mandrin 5 gegen ungewolltes Herausgleiten aus der Kanüle 3 gesichert ist.

## Patentansprüche

1. Kanülenansatzsystem (1) mit einem Kanülenansatz (2) und einem weiteren Ansatz (4), welche über eine Verdrehsicherung (14) lösbar miteinander verbunden sind,
**dadurch gekennzeichnet,**
**dass** einer der Ansätze mindestens einen radialen Vorsprung (8) aufweist und der andere Ansatz mindestens einen radialen Absatz (11) aufweist, wobei die Ansätze axial zusammenführbar sind und durch Verdrehen in einen lösbaren, bajonettartigen Hintergriff des Vorsprungs mit dem Absatz bringbar sind, wobei das Verdrehen in die Hintergriffstellung die Verdrehsicherung aktiviert.

2. Kanülenansatzsystem nach Anspruch 1,
**dadurch gekennzeichnet,**
**dass** die Verdrehsicherung (14) als Raste ausgebildet ist.

3. Kanülenansatzsystem nach mindestens einem der vorhergehenden Ansprüche,
**dadurch gekennzeichnet,**
**dass** die Verdrehsicherung (14) axial federnd ausgebildet ist.

4. Kanülenansatzsystem nach mindestens einem der vorhergehenden Ansprüche,
**dadurch gekennzeichnet,**
**dass** die Verdrehsicherung (14) einen Federsteg (12) aufweist, welcher mit Hilfe einer Nut (13) gebildet ist, die in geringem Abstand zur steckseitigen Stirnseite (9) des Ansatzes, benachbart zu dem Federsteg (12) vorgesehen ist.

5. Kanülenansatzsystem nach Anspruch 4,
**dadurch gekennzeichnet,**
**dass** der Federsteg (12) in die steckseitige Stirnfläche (9) des Ansatzes integriert ist.

6. Kanülenansatzsystem nach wenigstens einem der vorhergehenden Ansprüche,
**dadurch gekennzeichnet,**
**dass** der weitere Ansatz als Mandrin- oder Kanülenansatz ausgebildet ist.

## Geänderte Patentansprüche

### Geänderte Patentansprüche gemäss Regel 86(2) EPÜ.

**1.** Kanülenansatzsystem (1) mit einem Kanülenansatz (2) und einem weiteren Ansatz (4), welche über eine Verdrehsicherung (14) lösbar miteinander verbunden sind, wobei einer der Ansätze mindestens einen radialen Vorsprung (8) aufweist und der andere Ansatz mindestens einen radialen Absatz (11) aufweist, und die Ansätze axial zusammenführbar und durch Verdrehen in einen lösbaren, bajonettartigen Hintergriff des Vorsprungs mit dem Absatz bringbar sind, wobei das Verdrehen in die Hintergriffstellung die separate Verdrehsicherung aktiviert, und die Verdrehsicherung an einem der Ansatzstücke (4) einen axial federnden Federsteg (12) mit einer Raste (10) aufweist, welcher mit Hilfe einer etwa quer zur Axialrichtung verlaufenden Nut (13) gebildet ist, wobei die Raste (10) in der Hintergriffstellung in eine entsprechende Ausnehmung (7) an dem anderen Ansatz (2) einrastet,
**dadurch gekennzeichnet,**
**dass** der Federsteg (12) etwa quer zu der Zusammenführungsrichtung der Ansätze (2, 4) angeordnet ist, und der Federsteg (12) einteilig mit der steckseitigen Stirnfläche (9) des Ansatzes (4) ausgebildet ist, und die Nut (13) benachbart zur Raste (10) und in geringem Abstand zur steckseitigen Stirnseite (9) des Ansatzstücks (4) ausgebildet ist.

**2.** Kanülenansatzsystem nach Anspruch 1,
**dadurch gekennzeichnet,**
**dass** der weitere Ansatz als Mandrin- oder Kanülenansatz ausgebildet ist.
